# EUROPÄISCHE PATENTSCHRIFT

(11) **EP 0 980 874 B1**
(45) Veröffentlichungstag und Bekanntmachung des Hinweises auf die Patenterteilung: **02.05.2002**
(21) Anmeldenummer: 99115386.7
(22) Anmeldetag: 11.08.1998
(51) Int. Cl.: C07K 14/62

(54) **Verbessertes Verfahren zur Gewinnung von Insulinvorläufern mit korrekt verbundenen Cystinbrücken**
Process for production of insulin precursors having correctly linked cystein bridges
Procédé de préparation de proinsuline à ponts de cystine liés correctement

(30) Priorität: 18.08.1997 DE 19735711
(43) Veröffentlichungstag der Anmeldung: 23.02.2000
(62) Teilanmeldung aus: 98115048.5
(73) Patentinhaber: Aventis Pharma Deutschland GmbH, 65929 Frankfurt am Main (DE)
(72) Erfinder: Rubröder, Franz-Josef, Dr., 65606 Vilmar (DE); Keller, Reinhold, Dr., 65812 Bad Soden (DE)

(56) Entgegenhaltungen:
- EP-A- 0 600 372
- EP-A- 0 668 292
- EP-A- 0 906 918
- WO-A-96/20724

## Beschreibung

Die vorliegende Erfindung betrift ein verbessertes Verfahren zur Gewinnung eines Vorläufers von Insulinen oder Insulinderivaten mit korrekt verbundenen Cystinbrücken in Gegenwart von Cystein oder Cysteinhydrochlorid und von einem chaotropen Hilfsstoff.

Humaninsulin ist ein Protein mit zwei Aminosäureketten mit zusammen 51 Aminosäureresten. In den beiden Aminosäureketten kommen 6 Cysteinreste vor, wobei je zwei Cysteinreste über eine Disulfidbrücke untereinander verbunden sind. in biologisch aktivem Humaninsulin sind die A- und B-Ketten über zwei Cystinbrücken miteinander verbunden, und eine weitere Cystinbrücke kommt in der A-Kette vor. Innerhalb eines Humaninsulinmoleküls gibt es statistisch gesehen 15 Möglichkeiten zur Ausbildung von Disulfidbrücken. Im biologisch wirksamen Humaninsulin kommt nur eine der 15 Möglichkeiten vor. Die folgenden Cysteinreste sind im Humaninsulin miteinander verknüpft:
A 6-A11
A 7-B 7
A20-B 19

Die Buchstaben A und B stehen für die jeweilige Insulinaminosäurekette, die Zahl für die Position des Aminosäurerestes, die vom Amino- zum Carboxylende der jeweiligen Aminosäurekette gezählt wird. Disulfidbrücken können auch zwischen zwei Humaninsulinmolekülen ausgebildet werden, so daß leicht unübersehbar viele verschiedene Disulfidbrücken entstehen können.

Ein bekanntes Verfahren zur Herstellung von Humaninsulin beruht auf der Verwendung von humanem Proinsulin. Humanes Proinsulin ist ein Protein mit einer linearen Aminosäurekette aus 86 Aminosäureresten, wobei die B- und A-Ketten des Humaninsulins über ein C-Peptid mit 35 Aminosäureresten miteinander verbunden sind. Die Ausbildung der in Humaninsulin vorkommenden Disulfidbrücken erfolgt über ein Zwischenprodukt, wobei die Cysteinreste des Humaninsulins mit einer Schwefelschutzgruppe, z.B. einer S-Sulfonat (-S-SO₃⁻)-Gruppe versehen sind (EP 0 037 255). Ferner ist ein Verfahren zur Gewinnung von Proinsulin mit korrekt verbundenen Cystinbrücken bekannt (Biochemistry, 60, (1968), Seiten 622 bis 629), das von Proinsulin gewonnen aus Schweine-pankreas ausgeht, bei dem die Cysteinreste als Thiolreste (-SH) vorliegen. Unter dem Begriff "korrekt verbundenen Cystinbrücken" versteht man die Disulfidbrücken, die in biologisch aktivem Insulin aus Säugetieren vorkommen.

Gentechnische Verfahren erlauben es, Vorläufer von Insulin oder Insulinderivaten, im besonderen menschliches Proinsulin oder Proinsulin, das eine von Humaninsulin abweichende Aminosäuresequenz und/oder Aminosäurekettenlänge hat, in Mikroorganismen herzustellen. Die von gentechnisch veränderten Escherichia-coli-Zellen hergestellten Proinsuline haben keine korrekt verbundenen Cystinbrücken. Ein Verfahren zur Gewinnung von Humaninsulin mit E. coli (EP 0 055 945) beruht auf folgenden Verfahrensschritten:
Fermentation der Mikroorganismen - Zellaufschluß - Isolierung des Fusionproteins - Halogencyanspaltung des Fusionproteins - Isolierung des Spaltprodukts mit der Proinsulinsequenz - Schutz der Cysteinreste von Proinsulin durch S - Sulfonat-Gruppen - Chromatographische Reinigung des S-Sulfonats - Ausbildung der korrekt verbundenen Cystinbrücken - Entsalzen des Proinsulins - Chromatographische Reinigung des Proinsulins mit korrekt verbundenen Cystinbrücken - Konzentrierung der Proinsulinlösung - Chromatographische Reinigung der konzentrierten Proinsulinlösung - Enzymatische Spaltung des Proinsulins zur Gewinnung von Humaninsulin - Chromatographische Reinigung des entstandenen Humaninsulins.

Nachteile dieses Verfahrens sind die Anzahl der Verfahrensschritte und die Verluste bei den Reinigungsschritten, die zu einer geringen Ausbeute an Insulin führen. Wegen des vielstufigen Verfahrensweges müssen erhebliche Verluste in Kauf genommen werden. Von der Stufe des isolierten Fusionproteins über Halogencyanspaltung, Sulfitolyse und Reinigung des Proinsulins muß mit einem bis zu 40%igem Verlust von Proinsulin gerechnet werden (EP 0 055 945). Ähnlich hohe Verluste können im Verlauf der nachfolgenden Reinigungsschritte bis zum Endprodukt auftreten.

Ausbeutesteigerungen bei der gentechnischen Herstellung von Humaninsulin oder Insulinderivaten lassen sich dann erzielen, wenn die Zahl der notwendigen Verfahrensschritte wesentlich verringert werden kann.

Aus der EP 0 600 372 A1 (bzw. US 5,473,049) und der EP 0 668 292 A2 ist ein entsprechend verbessertes Verfahren zur Gewinnung von Insulinen oder Insulinderivaten bekannt, bei dem der Insulinvorläufer oder Vorläufer des Insulinderivates, dessen Cystinbrücken nicht korrekt verknüpft vorliegen, in Gegenwart eines Mercaptans, beispielsweise Cystein, und von mindestens einem chaotropen Hilfsstoff, beispielsweise Harnstoff oder Guanidinhydrochlorid, zu einem Insulinvorläufer bzw. Vorläufer des Insulinderivats mit korrekt verbundenen Cystinbrücken umgesetzt wird. Bei dem bekannten Verfahren werden diese Proteine zunächst in wäßrigen Lösungen eines chaotropen Hilfsmittels oder von Mischungen von verschiedenen chaotropen Hilfsmitteln in sehr niedriger Konzentration gelöst. Anschließend wird die Proteinmischung mit einer wäßrigen Mercaptanlösung gemischt.

Überraschenderweise wurde nun gefunden, daß sich die Ausbeuten an korrekt gefalteten Vorläufern von Insulinen oder Insulinderivaten dadurch erhöhen und die Reaktionszeiten für den Faltungsprozeß dadurch verringern lassen, daß man den Vorläufer nicht in einem ersten Schritt mittels des chaotropen Hilfsstoffs in Lösung bringt, sondern daß man zunächst in die wäßrige Suspension des Vorläufers das Mercaptan, nämlich Cystein oder Cysteinhydrochlorid, einträgt und erst in einem nachfolgenden Schritt die Lösung des Vorläufers durch Eintrag in eine wäßrige Lösung des chaotropen Hilfsstoffs und schließlich die korrekte Faltung des Vorläufers durch Verdünnung des Gemisches auf eine bevorzugte Cystein- bzw. Cysteinhydrochloridkonzentration unter Eintrag des Gemisches in eine entsprechende Menge an Wasser bewirkt.

Demgemäß betrifft die vorliegende Erfindung ein Verfahren zur Gewinnung eines Vorläufers von Insulinen oder Insulinderivaten mit korrekt verbundenen Cystinbrücken in Gegenwart von Cystein oder Cysteinhydrochlorid und von einem chaotropen Hilfsstoff, dadurch gekennzeichnet, daß man nacheinander die folgenden Schritte durchführt:
(a) Versetzen einer wäßrigen Suspension des Vorläufers von Insulinen oder Insulinderivaten mit einer Menge an Cystein oder Cysteinhydrochlorid, die 1 bis 15 SH-Reste des Cysteins oder Cysteinhydrochlorids pro Cysteinrest des Vorläufers ergibt,
(b) Eintragen der cystein- oder cysteinhydrochloridhaltigen Suspension des Vorläufers in eine 4 bis 9 molare Lösung des chaotropen Hilfsstoffs bei einem pH-Wert von ungefähr 8 bis ungefähr 11,5 und einer Temperatur von ungefähr 15 bis ungefähr 55 °C, Halten des erhaltenen Gemischs für etwa 10 bis 60 Minuten auf dieser Temperatur und
(c) Eintragen des Gemischs bei einem pH-Wert von ungefähr 8 bis ungefähr 11,5 und einer Temperatur von ungefähr 5 bis ungefähr 30 °C in eine Menge an Wasser, welche eine Verdünnung der Konzentration des Cysteins oder des Cysteinhydrochlorids in dem Gemisch auf etwa 1 bis 5 mM und des chaotropen Hilfsstoffs auf 0,2 bis 1,0 M ergibt.

Vorzugsweise ist das Verfahren dadurch gekennzeichnet, daß
in Schritt (a) die Menge an Cystein oder Cysteinhydrochlorid einer Menge entspricht, die 1 bis 6 SH-Reste des Cysteins oder Cysteinhydrochlorids pro Cysteinrest des Vorläufers ergibt,
in Schritt (b) das Eintragen der cystein- oder cysteinhydrochloridhaltigen Suspension des Vorläufers in eine 4 bis 9 molare Lösung des chaotropen Hilfsstoffs bei einem pH-Wert von 8 bis 11 und einer Temperatur von 30 bis 45 °C, das Halten des erhaltenen Gemischs für 20 bis 40 Minuten auf dieser Temperatur und
in Schritt (c) das Eintragen des Gemischs bei einem pH-Wert von 8 bis 11 und bei einer Temperatur von 15 bis 20 °C in eine Menge an Wasser, welche eine Verdünnung der Konzentration des Cysteins oder des Cysteinhydrochlorids in dem Gemisch auf etwa 1 bis 5 mM und eine Konzentration des chaotropen Hilfsstoffs von 0,2 bis 1,0 M ergibt, erfolgt.

Chaotrope Hilfsstoffe sind Verbindungen, die in wäßriger Lösung Wasserstoffbrückenbindungen brechen, beispielsweise Ammoniumsulfat, Guanidinhydrochlorid, Ethylencarbonat, Thiocyanat, Dimethylsulfoxid und Harnstoff.

Bei dem Verfahren gemäß der vorliegenden Erfindung wird als chaotroper Hilfsstoff bevorzugt Guanidin, Guanidinhydrochlorid oder besonders bevorzugt Harnstoff eingesetzt.

Die Konzentration des chaotropen Hilfsstoffs beträgt in Schritt (b) des erfindungsgemäßen Verfahrens vorzugsweise 7,0 bis 9M, die Temperatur in Schritt (b) vorzugsweise 40°C und der pH-Wert in Schritt (b) vorzugsweise 10 bis 11.

Bei dem erfindungsgemäßen Verfahren beträgt der pH-Wert in Schritt (c) vorzugsweise 10 bis 11. In Schritt (c) des Verfahrens gemäß der vorliegenden Erfindung wird die Menge an Wasser, in welche das Gemisch eingetragen wird, vorzugsweise so gewählt, daß diese eine Verdünnung der Cystein- oder Cystinhydrochloridkonzentration in dem Gemisch auf 2,5 bis 3 mM und eine Konzentration des chaotropen Hilfsstoffs von 0,5 M ergibt.

Besonders bevorzugt ist das erfindungsgemäße Verfahren dadurch gekennzeichnet, daß die Konzentration des chaotropen Hilfsstoffs in Schritt (b) etwa 8 M, die Temperatur in Schritt (b) etwa 40 °C, der pH-Wert in Schritt (b) etwa 10,6, der pH-Wert in Schritt (c) etwa 10,6 beträgt und in Schritt (c) die Menge an Wasser eine Verdünnung der Konzentration des Cysteins oder des Cysteinhydrochlorids in dem Gemisch auf etwa 2,5 bis 3 mM und eine Konzentration des chaotropen Hilfsstoffs von 0,5 M ergibt.

Das Ergebnis des Verfahrens gemäß der vorliegenden Erfindung ist ein Vorläufer von Insulinen oder Insulinderivaten, im besonderen ein Proinsulin, dessen Cystinbrücken korrekt verbunden sind.

Insulinderivate sind Derivate von natürlich vorkommenden Insulinen, nämlich Humaninsulin (s. SEQ ID NO 1 = A-Kette von Humaninsulin; s. SEQ ID NO 2 = B-Kette von Humaninsulin, Sequenzprotokoll) oder tierischen Insulinen, welche sich durch Substitution wenigstens eines natürlich auftretenden Aminosäurerestes und/oder Addition wenigstens eines Aminosäurerestes und/oder organischen Restes von dem entsprechenden, ansonst gleichen natürlich vorkommenden Insulin unterscheiden.

Aus dem mit Hilfe des Verfahrens gemäß der vorliegenden Erfindung gewonnenen Vorläufer der Insuline bzw. Insulinderivate mit korrekt verbundenen Cystinbrücken kann schließlich nach dem in der EP 0 600 372 A1 (bzw. US 5,473,049) oder in der EP 0 668 292 A2 beschriebenen Verfahren durch enzymatische Spaltung mittels Trypsin oder eines trypsinähnlichen Enzyms und gegebenenfalls zusätzlich mittels Carboxypeptidase B und anschließender Reinigung an einem Adsorberharz ein Insulin oder ein Insulinderivat mit korrekt verbundenen Cystinbrücken hergestellt werden.

Das aus dem Vorläufer herstellbare Insulin oder Insulinderivat läßt sich vorzugsweise durch Formel I beschreiben 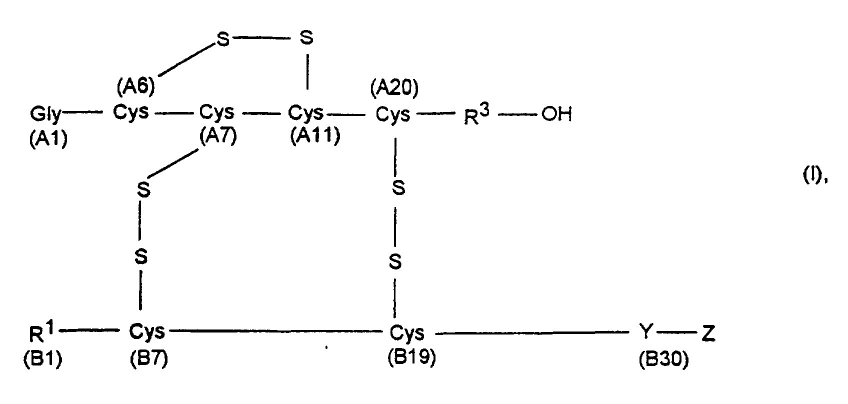 worin bedeuten
- Y: ein genetisch kodierbarer Aminosäurerest,
- Z: a) ein Aminosäurerest aus der Gruppe His, Arg oder Lys,
b) ein Peptid mit 2 oder 3 Aminosäureresten, enthaltend den Aminosäurerest Arg oder Lys am Carboxylende des Peptids,
c) ein Peptid mit 2 - 35 genetisch kodierbaren Aminosäuren, enthaltend 1 bis 5 Histidinreste, oder
d) OH,
- R¹: ein Phenylalaninrest (Phe) oder eine kovalente Bindung,
- R³: ein genetisch kodierbarer Aminosäurerest,
wobei die zur Vereinfachung der Formel I nicht gezeigten Reste A2 - A20 der Aminosäuresequenz der A-Kette von Humaninsulin, tierischen Insulin oder einem Insulinderivat und die zur Vereinfachung der Formel I nicht gezeigten Reste B2 - B29 der Aminosäuresequenz der B-Kette von Humaninsulin, tierischen Insulin oder einem Insulinderivat entsprechen.

Die Aminosäuresequenz von Peptiden und Proteinen wird vom N-terminalen Ende der Aminosäurekette an bezeichnet. Die in Formel I in Klammern gemachten Angaben, z.B. A6, A20, B1, B7 oder B19, entsprechen der Position von Aminosäureresten in den A- oder B-Ketten des Insulins.

Für den Begriff "genetisch kodierbarer Aminosäurerest" stehen die Aminosäuren Gly, Ala, Ser, Thr, Val, Leu, Ile, Asp, Asn, Glu, Gln, Cys, Met, Arg, Lys, His, Tyr, Phe, Trp, Pro und Selenocystein.

Für die Begriffe "Reste A2 - A20" und "Reste B2 - B 29" von tierischem Insulin werden beispielsweise die Aminosäuresequenzen von Insulin aus Rindern, Schweinen oder Hühnern verstanden. Der Begriff "Reste A2 - A20" und "B2 - B29" von Insulinderivaten steht für die entsprechenden Aminosäuresequenzen von Humaninsulin, die durch den Austausch von Aminosäuren durch andere genetisch kodierbare Aminosäuren gebildet werden.

Die A-Kette von Humaninsulin hat beispielsweise folgende Sequenz (SEQ ID NO.: 1):

Gly Ile Val Glu Gln Cys Cys Thr Ser Ile Cys Ser Leu Tyr Gln Leu
Glu Asn Tyr Cys Asn.

Die B-Kette von Humaninsulin hat folgende Sequenz (SEQ ID NO.: 2 ):

Phe Val Asn Gln His Leu Cys Gly Ser His Leu Val Glu Ala Leu Tyr
Leu Val Cys Gly Glu Arg Gly Phe Phe Tyr Thr Pro Lys Thr.

Dabei ist in Formel IR³ Asparagin (Asn), R¹ Phenylalanin (Phe), Y Threonin (Thr) und Z OH.

Das Verfahren gemäß der vorliegenden Erfindung ist dementsprechend besonders zur Gewinnung eines Vorläufers von Insulinen oder Insulinderivaten mit der allgemeinen Formel II, dessen Cystinbrücken (in Formel II nicht gezeigt) korrekt gefaltet sind,

R²-R¹-(B2-B29)-Y-X-Gly-(A2-A20)-R³ (II)

geeignet,
worin bedeuten
- R²: a) ein Wasserstoffatom,
b) ein Aminosäurerest aus der Gruppe Lysin (Lys) oder Arginin (Arg) oder
c) ein Peptid mit 2 bis 45 Aminosäureresten, enthaltend den Aminosäurerest Lysin (Lys) oder Arginin (Arg) am Carboxylende des Peptids
- R¹: ein Phenylalaninrest (Phe) oder eine kovalente Bindung,
- (B2-B29): die Aminosäurereste in den Positionen B2 bis B29 der B-Kette von Humaninsulin, tierischem Insulin oder einem gegebenenfalls in einer oder mehrerer dieser Positionen variierten Insulinderivat,
- Y: ein genetisch kodierbarer Aminosäurerest,
- X: a) ein Aminosäurerest aus der Gruppe Lysin (Lys) oder Arginin (Arg),
b) ein Peptid mit 2 bis 35 Aminosäureresten, enthaltend den Aminosäurerest Lysin (Lys) oder Arginin (Arg) am N-terminalen Ende und amCarboxylende des Peptids, oder
c) ein Peptid mit 2 bis 35 genetisch kodierbaren Aminosäuren, enthaltend 1 bis 5 Histidinreste,
- (A2-A20): die Aminosäurereste in den Positionen A2 bis A20 der B-Kette von Humaninsulin, tierischem Insulin oder einem gegebenenfalls in einer oder mehrerer dieser Positionen variierten Insulinderivat und
- R³: ein genetisch kodierbarer Aminosäurerest.

### 1. Vorzugsweise bedeuten in Formel II:

- R²: a) ein Wasserstoffatom oder
b) ein Peptid mit 2 bis 25 Aminosäureresten, enthaltend den Aminosäurerest Arginin (Arg) am Carboxylende des Peptids,
- R¹: ein Phenylalaninrest (Phe),
- (B2-B29): die Aminosäurereste in den Positionen 82 bis B29 der B-Kette von Humaninsulin,
- Y: ein Aminosäurerest aus der Gruppe Alanin (Ala), Threonin (Thr) oder Serin (Ser),
- X: der Aminosäurerest Arginin (Arg) oder ein Peptid mit der Aminosäuresequenz der C-Kette von Humaninsulin,
- (A2-A20): die Aminosäurereste in den Positionen A2 bis A20 der B-Kette von Humaninsulin und
- R³: ein Aminosäurerest aus der Gruppe Asparagin (Asn), Serin (Ser) oder Glycin (Gly).

Die C-Kette von Humaninsulin hat folgende Sequenz (SEQ ID NO.: 3):

Arg Arg Glu Ala Glu Asp Leu Gln Val Gly Gln Val Glu Leu Gly Gly
Gly Pro Gly Ala Gly Ser Leu Gln Pro Leu Ala Leu Glu Gly Ser Leu
Gln Lys Arg.

### 2. Vorzugsweise bedeuten in Formel II:

- R²: a) ein Wasserstoffatom oder
b) ein Peptid mit 2 bis 15 Aminosäureresten, an dessen Carboxylende sich ein Argininrest (Arg) befindet,
- R¹: ein Phenylalaninrest (Phe),
- (B2-B29): die Aminosäurereste in den Positionen B2 bis B29 der B-Kette von Humaninsulin,
- Y: ein Threoninrest (Thr),
- X: der Aminosäurerest Arginin (Arg) oder ein Peptid mit 2 bis 35 Aminosäureresten, wobei am Anfang und am Ende des Peptids zwei basische Aminosäurereste, insbesondere Arginin (Arg) und/oder Lysin (Lys) stehen,
- (A2-A20): die Aminosäurereste in den Positionen A2 bis A20 der B-Kette von Humaninsulin und
- R³: der Aminosäurerest Asparagin (Asn) oder Glycin (Gly).

Der Rest Z des Insulins oder des Insulinderivates der Formel I ist in der Regel Teil der Aminosäuresequenz von X des Vorläufers der Formel II und entsteht durch die Aktivität der Proteasen wie Trypsin, trypsinähnliches Enzym oder Carboxypeptidase B. Der Rest R³ ist der Aminosäurerest, der an Position A21 der A-Kette von Insulin steht. Der Rest Y ist der Aminosäurerest, der an Position B30 der B-Kette von Insulin steht.

Trypsin oder trypsinähnliche Enzyme sind Proteasen, die Aminosäureketten am Arginin- oder Lysinrest spalten.
Carboxypeptidase B ist eine Exoprotease, die basische Aminosäurereste wie Arg oder Lys, die am Carboxyterminalen Ende von Aminosäureketten stehen, abspaltet. (Kemmler et al., J. Biol. Chem. 246, Seiten 6786-6791).

Aus dem unter 1 genannten Vorläufer läßt sich beispielsweise ein Insulin bzw. Insulinderivat der Formel I mit korrekt verknüpften Cystinbrücken gewinnen, wobei Y, R¹, R², R³, A2-A20 und B2-B29 die unter 1 genannte Bedeutung haben und Z ein Argininrest (Arg), ein Peptidrest Arg-Arg oder -OH bedeutet.

Aus dem unter 2 genannten Vorläufer läßt sich beispielsweise ein Insulin bzw. Insulinderivat der Formel I mit korrekt verknüpften Cystinbrücken gewinnen, wobei Y, R¹, R², R³, A2-A20 und B2-B29 die unter 2 genannte Bedeutung haben und Z ein Argininrest (Arg), ein Peptidrest Arg-Arg oder Lys-Lys oder -OH bedeutet.

Der Vorläufer der Formel II kann mit Hilfe einer Vielzahl gentechnischer Konstrukte in Mikroorganismen gebildet werden (EP 0 489 780, EP 0 347 781, EP 0 453 969). Die gentechnischen Konstrukte werden in Mikroorganismen wie Escherichia coli oder Streptomyceten während der Fermentation exprimiert. Die gebildeten Proteine werden im Inneren der Mikroorganismen abgelagert (EP 0 489 780) oder in die Fermentationslösung ausgeschieden.

Für das erfindungsgemäße Verfahren können Vorläufer von Insulinen oder von Insulinderivaten der Formel II eingesetzt werden, die direkt nach dem Zellaufschluß noch mit einer Vielzahl von Proteinen, die aus der Fermentationslösung und aus den Mikroorganismen stammen, verunreinigt sind. Die Vorläufer der Formel II können aber auch in vorgereinigter Form beispielsweise nach einer Fällung oder einer chromatographischen Reinigung eingesetzt werden.

### Beispiel 1 (Vergleichsbeispiel, Stand der Technik)

Durch Fermentation von genetisch modifizierten Escherichia-coli-Zellen (EP 0 489 780) wird ein Fusionsprotein mit folgender Aminosäuresequenz hergestellt.

Proinsulinsequenz 1 (SEQ ID NO.: 4):

Ala Thr Thr Ser Thr Gly Asn Ser Ala Arg Phe Val Asn Gln His Leu
Cys Gly Ser His Leu Val Glu Ala Leu Tyr Leu Val Cys Gly Glu Arg
Gly Phe Phe Tyr Thr Pro Lys Thr Arg Arg Glu Ala Glu Asp Leu Gln
Val Gly Gln Val Glu Leu Gly Gly Gly Pro Gly Ala Gly Ser Leu Gln
Pro Leu Ala Leu Glu Gly Ser Leu Gln Lys Arg Gly Ile Val Glu Gln
Cys Cys Thr Ser Ile Cys Ser Leu Tyr Gln Leu Glu Asn Tyr Cys Asn

Proinsulinsequenz 1 entspricht der Formel II, dabei ist
- X: C-Peptid von Humaninsulin (SEQ ID NO.: 3)
- Y: Thr (B30),
- R¹: Phe (B1),
- R²: ein Peptid mit 10 Aminosäureresten,
- R³: Asn (A21) und
A2 - A20 die Aminosäuresequenz der A-Kette von Humaninsulin (Aminosäurereste 2 bis 20) und B2 - B29 die Aminosäuresequenz der B-Kette von Humaninsulin (Aminosäurereste 2 bis 29).

In den E.-coli-Zellen sammelt sich das exprimierte Fusionsprotein mit der Proinsulinsequenz 1 an und bildet Einschlußkörper. Nach Beendigung der Fermentation werden die Zellen durch Zentrifugation abgetrennt und durch übliche Hochdruckhomogenisation aufgeschlossen. Die freigesetzten Fusionsproteineinschlußkörperchen werden durch Zentrifugation isoliert.

20 kg der isolierten Fusionsproteineinschlußkörper (bezogen auf Trockenmasse nach Gefriertrocknung; der Anteil des insulinhaltigen Fusionsproteins wird mit Hilfe der HPLC bestimmt; er beträgt 50 %) mit der Proinsulinsequenz 1 werden in 550 I einer 8 M Harnstoff-Lösung bei pH 10,6 gelöst. Gegebenenfalls wird nach Zentrifugation geringer Mengen Trübstoffe die klare Lösung in 9000 I einer wäßrigen Cysteinlösung (5 kg Cysteinhydrochloridhydrat) bei einem pH-Wert von 10,6 und einer Temperatur von 4°C eingerührt.
Nach Abschluß der Faltungsreaktion nach ca. 24 h wird mit Hilfe der analytischen HPLC der Gehalt an Proinsulinsequenz I mit korrekt verbundenen Cystinbrücken im Reaktionsansatz mit 3,0 kg entsprechend einem Umsatz von 30 % bestimmt.

Die 9500 I Lösung wird mit 1 N HCI auf einen pH von 5,0 eingestellt und separiert. Dann erfolgt durch Zugabe von 1 N Natronlauge die Einstellung von pH 9. Es werden 3 g Trypsin in die Lösung eingetragen. Es entsteht 1,25 kg eines Insulins mit 2 carboxyterminalen Argininresten gemäß HPLC-Messung.
Nach Spaltung mit Carboxypeptidase B entsteht Humaninsulin, das mit Hilfe chromatorgraphischer Methoden noch gereinigt wird.
Humaninsulin entspricht der Formel I, dabei ist
- Y: Thr (B30),
- Z: OH,
- R¹: Phe(B1),
- R³: Asn (A21) und
A2 - A20 die Aminosäuresequenz der A-Kette von Humaninsulin (aminosäurereste 2 bis 20) und B2-B29 die Aminosäuresequenz der B-Kette von Humaninsulin (Aminosäurereste 2 bis 29).

Humaninsulin 2 besteht aus der SEQ ID NO.: 1 und 2, die über korrekt gebundene Cystinbrücken miteinander verbunden sind.

Die Lösung wird wie in der EP 0 668 292 beschrieben mittels Adsorberharz aufkonzentriert und gereinigt. Das Eluat, das Insulin 2 enthält, kann nach Verdünnung mit Wasser und pH-Einstellung unmittelbar auf einer Chromatographiesäule weiter gereinigt werden.

### HPLC-Analyse

0,5 g Protein werden in 40 ml einer Lösung aus 6 M Guanidinhydrochlorid, 50 mM Tris, pH 8,5, 5 mM Ethylendiamintetraacetat (EDTA), 1 % 2-Mercaptoethanol, 10 mM Dithiothreitol bei 95°C für 2 min gelöst und dann bei 14000 g für 20 min zentrifugiert. Von dem klaren Überstand werden 0,02 ml auf eine Hochdruckflüssigchromatographiesäule aufgetragen.
- Säule:: ®Nucleogel RP 300-5/46 (Macherey & Nagel, Aachen, Deutschland)
- Gradient:: Puffer A: 0,1 % Trifluoressigsäure (TFA)
Puffer B: 0,09 % TFA in Acetonitril
- Temperatur:: 55 C
- Gesamtlaufzeit:: 40 min,

Der Gradient ist gekennzeichnet durch die folgende Mengen von Puffer B nach den entsprechenden Laufzeiten :
10 min 25%, 12 min 60 %, 13 min 90 %, 15 min 100 %.
   - Fluß:: 1 ml/min
   - Detektion:: 215 nm
Retentionszeit von
   - Insulin:: etwa 19 min

### Beispiel 2 (Verfahren gemäß der vorliegenden Erfindung)

Durch Fermentation von genetisch modifizierten Escherichia-coli-Zellen (EP 0 489 780) wird ein Fusionsprotein mit der in Beispiel 1 dargestellten Aminosäuresequenz hergestellt (Proinsulinsequenz 1. SEQ ID NO.: 4).

In den E.-coli-Zellen sammelt sich das exprimierte Fusionsprotein mit der Proinsulinsequenz 1 an und bildet Einschlußkörper. Nach Beendigung der Fermentation werden die Zellen durch Zentrifugation abgetrennt und durch übliche Hochdruckhomogenisation aufgeschlossen. Die freigesetzten Fusionsproteineinschlußkörper werden durch Zentrifugation isoliert.

Der wässrigen Fusionsproteinsuspension, die 40 kg Fusionsprotein (ermittelt durch Gefriertrocknung eines Aliquots) enthält, werden 5 kg Cysteinhydrochloridhydrat zugefügt.

Die Suspension wird (Der Anteil des insulinhaltigen Fusionproteins wird mit Hilfe der HPLC bestimmt. Er beträgt 50 %.) mit der Proinsulinsequenz 1 in 550 l einer 8 M Harnstoff-Lösung bei pH 10,2 bei 40°C gelöst. Die klare Lösung wird in 9000 I Wasser bei einem pH-Wert von 10,6 und einer Temperatur von 15°C eingerührt. Nach Abschluß der Faltungsreaktion nach ca. 5 Std wird mit Hilfe der analytischen HPLC der Gehalt an Proinsulinsequenz I mit korrekt verbundenen Cystinbrücken im Reaktionsansatz mit 10,0 kg entsprechend einem Umsatz von 50 % bestimmt.

Die 9500 I Lösung wird mit 1N HCI auf einen pH von 5,0 eingestellt und separiert. Dann erfolgt durch Zugabe von 1N Natronlauge die Einstellung von pH 9. Es werden 10 g Trypsin in die Lösung eingetragen. Es entstehen 4 kg eines Insulins mit 2 carboxyterminalen Argininresten. Nach Spaltung mit Carboxypeptidase B entsteht Humaninsulin (SEQ ID NO.: 1 und 2 mit korrekt verbundenen Cystinbrücken).

Die Lösung wird mittels Adsorberharz aufkonzentriert und gereinigt.

Das Eluat, das Humaninsulin enthält, kann nach Verdünnung mit Wasser und pH-Einstellung unmittelbar auf einer Chromatographiesäule weiter gereinigt werden.

### Beispiel 3 (Vergleichsbeispiel, Stand der Technik)

Durch Fermentation von genetisch modifizierten Escherichia-coli-Zellen (EP 0 489 780) wird ein Fusionsprotein mit folgender Aminosäuresequenz hergestellt.

Proinsulinsequenz 2 (SEQ ID NO.: 5):

Ala Thr Thr Ser Thr Gly Asn Ser Ala Arg Phe Val Asn Gln His Leu
Cys Gly Ser His Leu Val Glu Ala Leu Tyr Leu Val Cys Gly Glu Arg
Gly Phe Phe Tyr Thr Pro Lys Thr Arg Arg Glu Ala Glu Asp Leu Gln
Val Gly Gln Val Glu Leu Gly Gly Gly Pro Gly Ala Gly Ser Leu Gln
Pro Leu Ala Leu Glu Gly Ser Leu Gln Lys Arg Gly Ile Val Glu Gln
Cys Cys Thr Ser Ile Cys Ser Leu Tyr Gln Leu Glu Asn Tyr Cys Gly

Proinsulinsequenz 2 entspricht der Formel II, dabei ist
- X: C-Peptid von Humaninsulin (SEQ ID NO.: 3),
- Y: Thr (B30),
- R¹: Phe (B1),
- R²: ein Peptid mit 10 Aminosäureresten,
- R³: Gly (A21) und
A2 - A20 die Aminosäuresequenz der A-Kette von Humaninsulin (Aminosäurereste 2 bis 20) und B2 - B29 die Aminosäuresequenz der B-Kette von Humaninsulin (Aminosäurereste 2 bis 29).

In den E.-coli-Zellen sammelt sich das exprimierte Fusionsprotein mit der Proinsulinsequenz 2 an und bildet Einschlußkörper. Nach Beendigung der Fermentation werden die Zellen durch Zentrifugation abgetrennt und durch übliche Hochdruckhomogenisation aufgeschlossen. Die freigesetzten Fusionsproteineinschlußkörperchen werden durch Zentrifugation isoliert.

20 kg der isolierten Fusionsproteineinschlußkörper (bezogen auf Trockenmasse nach Gefriertrocknung; der Anteil des insulinhaltigen Fusionproteins wird mit Hilfe der HPLC bestimmt. Er beträgt 50 %.) mit der Proinsulinsequenz 2 werden in 550 I einer 8 M Harnstoff-Lösung bei pH 10,6 bei 20°C gelöst. Die klare Lösung wird in 9000 I einer wäßrigen Cysteinlösung (5 kg Cysteinhydrochloridhydrat) bei einem pH-Wert von 10,6 und einer Temperatur von 4°C eingerührt.
Nach Abschluß der Faltungsreaktion nach ca. 24 Std wird mit Hilfe der analytischen HPLC der Gehalt an Proinsulinsequenz 2 mit korrekt verbundenen Cystinbrücken im Reaktionsansatz mit 3,0 kg entsprechend einem Umsatz von 30 % bestimmt.

Die 9500 l Lösung wird mit 1N HCI auf einen pH von 5,0 eingestellt und separiert. Dann erfolgt durch Zugabe von 1 N Natronlauge die Einstellung von pH 9. Es werden 3 g Trypsin in die Lösung eingetragen. Es entstehen 0,98 kg eines Insulinderivats mit 2 carboxyterminalen Argininresten nach HPLC-Messung. Dieses Insulinderivat entspricht der Formel I, wobei
- Y: Thr (B30),
- Z: Arg-Arg,
- R¹: Phe (B1),
- R³: Gly (A21) und
A2 - A20 die Aminosäuresequenz der A-Kette von Humaninsulin (aminosäurereste 2 bis 20) und B2-B29 die Aminosäuresequenz der B-Kette von Humaninsulin (Aminosäurereste 2 bis 29) bedeuten, und besteht aus den SEQ ID NO.: 6 und 7, die über korrekt gebundene Cystinbrücken miteinander verbunden sind.

Die Lösung wird mittels Adsorberharz aufkonzentriert und gereinigt.

Das Eluat, welches das Insulinderivat enthält, kann nach Verdünnung mit Wasser und pH-Einstellung unmittelbar auf einer Chromatographiesäule weiter gereinigt werden.

### Beispiel 4 (Verfahren gemäß der vorliegenden Erfindung)

Durch Fermentation von genetisch modifizierten Escherichia-coli-Zellen (EP 0 489 780) wird das Fusionsprotein mit der Proinsulinsequenz 2 (SEQ ID NO.: 5) gemäß Beispiel 3 hergestellt.

In den E.-coli-Zellen sammelt sich das exprimierte Fusionsprotein mit der Proinsulinsequenz 2 an und bildet Einschlußkörper. Nach Beendigung der Fermentation werden die Zellen durch Zentrifugation abgetrennt und durch übliche Hochdruckhomogenisation aufgeschlossen. Die freigesetzten Fusionsproteineinschlußkörper werden durch Zentrifugation isoliert.

Der wässrigen Fusionsproteinsuspension, die 40 kg Fusionsprotein (Ermittelt durch Gefriertrocknung eines Aliquots) enthalten, werden 5 kg Cysteinhydrochloridhydrat zugefügt.

Die Suspension wird (Der Anteil des insulinhaltigen Fusionproteins wird mit Hilfe der HPLC bestimmt. Er beträgt 50 %.) mit der Proinsulinsequenz 2 in 550 I einer 8 M Harnstoff-Lösung bei pH 10,2 bei 40°C gelöst. Die klare Lösung wird in 9000 I Wasser bei einem pH-Wert von 10,6 und einer Temperatur von 15°C eingerührt. Nach Abschluß der Faltungsreaktion nach ca. 5 Std wird mit Hilfe der analytischen HPLC der Gehalt an Proinsulinsequenz I mit korrekt verbundenen Cystinbrücken im Reaktionsansatz mit 10,0 kg entsprechend einem Umsatz von 50 % bestimmt.

Die 9500 l Lösung wird mit 1N HCI auf einen pH von 5,0 eingestellt und separiert. Dann erfolgt durch Zugabe von 1 N Natronlauge die Einstellung von pH 9. Es werden 10 g Trypsin in die Lösung eingetragen. Es entstehen 2,8 kg des Insulinderivats (HPLC Messung), das aus den Sequenzen SEQ ID NO.: 6 und 7 besteht, die über korrekt gebundene Cystinbrücken miteinander verknüpft sind.

Die Lösung wird mittels Adsorberharz aufkonzentriert und gereinigt.
Das Eluat, welches das Insulinderivat enthält, kann nach Verdünnung mit Wasser und pH-Einstellung unmittelbar auf einer Chromatographiesäule weiter gereinigt werden.

## Patentansprüche

1. Verfahren zur Herstellung von Insulinen oder von einem Insulinderivat mit korrekt verbundenen Cystinbrücken aus einem Vorläufer des Insulin oder des Insulinderivats, wobei der Vorläufer in Gegenwart von Cystein oder Cysteinhydrochlorid und von einem chaotropen Hilfsstoff einem Faltungsprozeß unterworfen wird, wonach aus dem Vorläufer durch enzymatische Spaltung mittels Trypsin oder eines trypsinähnlichen Enzyms und gegebenenfalls zusätzlich mittels Carboxypeptidase B und anschließender Reinigung an einem Adsorberharz ein Insulin oder ein Insulinderivat mit korrekt verbundenen Cystinbrücken gewonnen wird, **dadurch gekennzeichnet, daß** man zur Ausführung des Faltungsprozeß nacheinander die folgenden Schritte durchführt:
(a) Versetzen einer wäßrigen Suspension des Vorläufers des Insulins oder des Insulinderivats mit einer Menge an Cystein oder Cysteinhydrochlorid, die 1 bis 15 SH-Reste des Cysteins oder Cysteinhydrochlorids pro Cysteinrest des Vorläufers ergibt,
(b) Eintragen der cystein- oder cysteinhydrochloridhaltigen Suspension des Vorläufers in eine 4 bis 9 molare Lösung des chaotropen Hilfsstoffs bei einem pH-Wert von ungefähr 8 bis ungefähr 11,5 und einer Temperatur von ungefähr 15 bis ungefähr 55 °C, Halten des erhaltenen Gemischs für etwa 10 bis 60 Minuten auf dieser Temperatur und
(c) Eintragen des Gemischs bei einem pH-Wert von ungefähr 8 bis ungefähr 11,5 und einer Temperatur von ungefähr 5 bis ungefähr 30 °C in eine Menge an Wasser, welche eine Verdünnung der Konzentration des Cysteins oder des Cysteinhydrochlorids in dem Gemisch auf etwa 1 bis 5 mM und des chaotropen Hilfsstoffs auf 0,2 bis 1,0 M ergibt.

2. Verfahren nach Anspruch 1, **dadurch gekennzeichnet, daß**
in Schritt (a) die Menge an Cystein oder Cysteinhydrochlorid einer Menge entspricht, die 1 bis 6 SH-Reste des Cysteins oder Cysteinhydrochlorids pro Cysteinrest des Vorläufers ergibt,
in Schritt (b) das Eintragen der cystein- oder cysteinhydrochloridhaltigen Suspension des Vorläufers in eine 4 bis 9 molare Lösung des chaotropen Hilfsstoffs bei einem pH-Wert von 8 bis 11 und einer Temperatur von 30 bis 45 °C, das Halten des erhaltenen Gemischs für 20 bis 40 Minuten auf dieser Temperatur und
in Schritt (c) das Eintragen des Gemischs bei einem pH-Wert von 8 bis 11 und bei einer Temperatur von 15 bis 20 °C in eine Menge an Wasser, welche eine Verdünnung der Konzentration des Cysteins oder des Cysteinhydrochlorids in dem Gemisch auf etwa 1 bis 5 mM ergibt und eine Konzentration des chaotropen Hilfsstoffs von 0,2 bis 1,0 M, erfolgt.

3. Verfahren nach Anspruch 1 oder 2, **dadurch gekennzeichnet, daß** der chaotrope Hilfsstoff Guanidin oder Guanidinhydrochlorid ist.

4. Verfahren nach Anspruch 1 oder 2, **dadurch gekennzeichnet, daß** der chaotrope Hilfsstoff Harnstoff ist.

5. Verfahren nach einem oder mehreren der Ansprüche 1 bis 4, **dadurch gekennzeichnet, daß** die Konzentration des chaotropen Hilfsstoffs in Schritt (b) 7,0 bis 9 M beträgt.

6. Verfahren nach einem oder mehreren der Ansprüche 1 bis 5, **dadurch gekennzeichnet, daß** die Temperatur in Schritt (b) 40 °C beträgt.

7. Verfahren nach einem oder mehreren der Ansprüche 1 bis 6, **dadurch gekennzeichnet, daß** der pH-Wert in Schritt (b) 10 bis 11 beträgt.

8. Verfahren nach einem oder mehreren der Ansprüche 1 bis 7, **dadurch gekennzeichnet, daß** der pH-Wert in Schritt (c) 10 bis 11 beträgt.

9. Verfahren nach einem oder mehreren der Ansprüche 1 bis 8, **dadurch gekennzeichnet, daß** in Schritt (c) die Menge an Wasser eine Verdünnung der Konzentration des Cysteins oder des Cysteinhydrochlorids in dem Gemisch auf 2,5 bis 3 mM und eine Konzentration des chaotropen Hilfsstoffs auf 0,5 M ergibt.

10. Verfahren nach einem oder mehreren der Ansprüche 2 bis 9, **dadurch gekennzeichnet, daß** die Konzentration des chaotropen Hilfsstoffs in Schritt (b) 8 M, die Temperatur in Schritt (b) 40 °C, der pH-Wert in Schritt (b) 10,6, der pH-Wert in Schritt (c) 10,6 beträgt und in Schritt (c) die Menge an Wasser eine Verdünnung der Konzentration des Cysteins oder des Cysteinhydrochlorids in dem Gemisch auf 2,5 bis 3 mM und eine Konzentration des chaotropen Hilfsstoffs von 0,5 M ergibt.

11. Verfahren nach einem oder mehreren der Ansprüche 1 bis 10, **dadurch gekennzeichnet, daß** der Vorläufer der Insuline oder der Insulinderivate die Sequenz gemäß der allgemeinen Formel II aufweist
R²-R¹-(B2-B29)-Y-X-Gly-(A2-A20)-R³ (II),
worin bedeuten
R²
a) ein Wasserstoffatom,
b) ein Aminosäurerest aus der Gruppe Lysin (Lys) oder Arginin (Arg) oder
c) ein Peptid mit 2 bis 45 Aminosäureresten, enthaltend den Aminosäurerest Lysin (Lys) oder Arginin (Arg) am Carboxylende des Peptids
R¹ ein Phenylalaninrest (Phe) oder eine kovalente Bindung,
(B2-B29) die Aminosäurereste in den Positionen B2 bis B29 der B-Kette von Humaninsulin, tierischem Insulin oder einem gegebenenfalls in einer oder mehrerer dieser Positionen variierten Insulinderivat,
Y ein genetisch kodierbarer Aminosäurerest,
X
a) ein Aminosäurerest aus der Gruppe Lysin (Lys) oder Arginin (Arg) oder
b) ein Peptid mit 2 bis 35 Aminosäureresten, enthaltend den Aminosäurerest Lysin (Lys) oder Arginin (Arg) am N-terminalen Ende und amCarboxylende des Peptids, oder
c) ein Peptid mit 2 bis 35 genetisch kodierbaren Aminosäuren, enthaltend 1 bis 5 Histidinreste,
(A2-A20) die Aminosäurereste in den Positionen A2 bis A20 der B-Kette von Humaninsulin, tierischem Insulin oder einem gegebenenfalls in einer oder mehrerer dieser Positionen variierten Insulinderivat und
R³ ein genetisch kodierbarer Aminosäurerest.

12. Verfahren nach Anspruch 11, **dadurch gekennzeichnet, daß** in Formel II
R²
a) ein Wasserstoffatom oder
b) ein Peptid mit 2 bis 25 Aminosäureresten, enthaltend den Aminosäurerest Arginin (Arg) am Carboxylende des Peptids,
R¹ ein Phenylalaninrest (Phe),
(B2-B29) die Aminosäurereste in den Positionen B2 bis B29 der B-Kette von Humaninsulin,
Y ein Aminosäurerest aus der Gruppe Alanin (Ala), Threonin (Thr) oder Serin (Ser),
X der Aminosäurerest Arginin (Arg) oder ein Peptid mit der Aminosäuresequenz der C-Kette von Humaninsulin,
(A2-A20) die Aminosäurereste in den Positionen A2 bis A20 der B-Kette von Humaninsulin und
R³ ein Aminosäurerest aus der Gruppe Asparagin (Asn), Serin (Ser) oder Glycin (Gly)
bedeuten.

13. Verfahren nach Anspruch 11, **dadurch gekennzeichnet, daß** in Formel II
R²
a) ein Wasserstoffatom oder
b) ein Peptid mit 2 bis 15 Aminosäureresten, an dessen Carboxylende sich ein Argininrest (Arg) befindet,
R¹ ein Phenylalaninrest (Phe),
(B2-B29) die Aminosäurereste in den Positionen B2 bis B29 der B-Kette von Humaninsulin,
Y ein Threoninrest (Thr),
X der Aminosäurerest Arginin (Arg) oder ein Peptid mit 2 bis 35 Aminosäureresten, wobei am Anfang und am Ende des Peptids zwei basische Aminosäurereste, insbesondere Arginin (Arg) und/oder Lysin (Lys) stehen,
(A2-A20) die Aminosäurereste in den Positionen A2 bis A20 der B-Kette von Humaninsulin und
R³ der Aminosäurerest Asparagin (Asn) oder Glycin (Gly)
bedeuten.

14. Verfahren nach einem oder mehreren der Ansprüche 1 bis 13, **dadurch gekennzeichnet, daß** ein Insulin oder ein Insulinderivat der Formel I 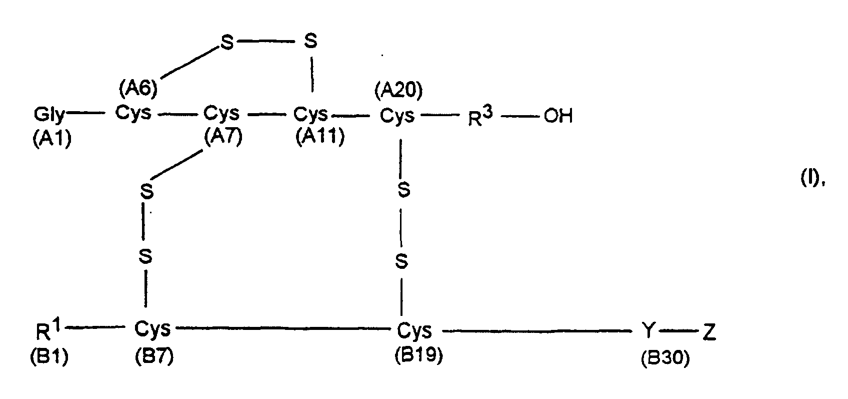 gewonnen wird, worin bedeuten
Y ein genetisch kodierbarer Aminosäurerest,
Z
a) ein Aminosäurerest aus der Gruppe His, Arg oder Lys,
b) ein Peptid mit 2 oder 3 Aminosäureresten, enthaltend den Aminosäurerest Arg oder Lys am Carboxylende des Peptids,
c) ein Peptid mit 2 - 35 genetisch kodierbaren Aminosäuren, enthaltend 1 bis 5 Histidinreste, oder
d) OH,
R¹ ein Phenylalaninrest (Phe)
R³ ein genetisch kodierbarer Aminosäurerest,
wobei die Reste A2 - A20 der Aminosäuresequenz der A-Kette von Humaninsulin, tierischen Insulin oder einem Insulinderivat und die Reste B2 - B29 der Aminosäuresequenz der B-Kette von Humaninsulin, tierischen Insulin oder einem Insulinderivat entsprechen.

15. Verfahren nach Anspruch 14, **dadurch gekennzeichnet, daß** in Formel I
Y Alanin (Ala), Threonin (Thr) oder Serin (Ser)
Z
a) Arginin (Arg)
b) ein Dipeptid mit 2 Argininresten (Arg-Arg)
c) OH,
R³ Asparagin (Asn), Serin (Ser), Glycin (Gly)
bedeuten.

16. Verfahren nach Anspruch 14, **dadurch gekennzeichnet, daß** in Formel I
Y Threonin (Thr)
Z
a) Arginin (Arg)
b) ein Dipeptid mit 2 Argininresten (Arg-Arg) oder 2 Lysinresten (Lys-Lys)
c) OH,
R³ Asparagin (Asn), Glycin (Gly)
bedeuten.

17. Verfahren nach Anspruch 15 oder 16 **dadurch gekennzeichnet, daß** in Formel I
Y Threonin (Thr)
Z OH,
R³ Asparagin (Asn)
bedeuten.

18. Verfahren nach Anspruch 15 oder 16, **dadurch gekennzeichnet, daß** in Formel I
Y Threonin (Thr)
Z ein Dipeptid mit 2 Argininresten (Arg-Arg)
R³ Glycin (Gly)
bedeuten.

## Claims

1. A process for preparing insulins or an insulin derivative having correctly bonded cystine bridges from a precursor of the insulin or of the insulin derivative, the precursor being subjected to a folding process in the presence of cysteine or cysteine hydrochloride and of a chaotropic auxiliary, after which an insulin or insulin derivative is obtained from the precursor by enzymatic cleavage by means of trypsin or of a trypsin-like enzyme and optionally additionally by means of carboxypeptidase B and subsequent purification on an adsorber resin which comprises successively carrying out the following steps in order to implement the folding process:
(a) mixing an aqueous suspension of the precursor of the insulin or of the insulin derivative with an amount of cysteine or cysteine hydrochloride which results in 1 to 15 SH residues of the cysteine or cysteine hydrochloride per cysteine residue of the precursor,
(b) introducing the cysteine- or cysteine hydrochloride-containing suspension of the precursor into a 4 to 9 molar solution of the chaotropic auxiliary at a pH of approximately 8 to approximately 11.5 and a temperature of approximately 15 to approximately 55°C, keeping the mixture obtained at this temperature for approximately 10 to 60 minutes and
(c) introducing the mixture at a pH of approximately 8 to approximately 11.5 and a temperature of approximately 5 to approximately 30°C into an amount of water which results in a dilution of the concentration of the cysteine or of the cysteine hydrochloride in the mixture to approximately 1 to 5 mM and of the chaotropic auxiliary to 0.2 to 1.0 M.

2. The process as claimed in claim 1, wherein
in step (a) the amount of cysteine or cysteine hydrochloride corresponds to an amount which results in 1 to 6 SH residues of the cysteine or cysteine hydrochloride per cysteine residue of the precursor,
in step (b) the cysteine- or cysteine hydrochloride-containing suspension of the precursor is introduced into a 4 to 9 molar solution of the chaotropic auxiliary at a pH of 8 to 11 and a temperature of 30 to 45°C and the mixture obtained is kept for 20 to 40 minutes at this temperature and
in step (c) the mixture is introduced at a pH of 8 to 11 and at a temperature of 15 to 20°C into an amount of water which results in a dilution of the concentration of the cysteine or of the cysteine hydrochloride in the mixture to approximately 1 to 5 mM and a concentration of the chaotropic auxiliary of 0.2 to 1.0 M.

3. The process as claimed in claim 1 or 2, wherein the chaotropic auxiliary is guanidine or guanidine hydrochloride.

4. The process as claimed in claim 1 or 2, wherein the chaotropic auxiliary is urea.

5. The process as claimed in one or more of claims 1 to 4, wherein the concentration of the chaotropic auxiliary in step (b) is 7.0 to 9 M.

6. The process as claimed in one or more of claims 1 to 5, wherein the temperature in step (b) is 40°C.

7. The process as claimed in one or more of claims 1 to 6, wherein the pH in step (b) is 10 to 11.

8. The process as claimed in one or more of claims 1 to 7, wherein the pH in step (c) is 10 to 11.

9. The process as claimed in one or more of claims 1 to 8, wherein in step (c) the amount of water results in a dilution of the concentration of the cysteine or of the cysteine hydrochloride in the mixture to 2.5 to 3 mM and a concentration of the chaotropic auxiliary to 0.5 M.

10. The process as claimed in one or more of claims 2 to 9, wherein the concentration of the chaotropic auxiliary in step (b) is 8 M, the temperature in step (b) is 40°C, the pH in step (b) is 10.6, the pH in step (c) is 10.6 and in step (c) the amount of water results in a dilution of the concentration of the cysteine or of the cysteine hydrochloride in the mixture to 2.5 to 3 mM and a concentration of the chaotropic auxiliary of 0.5 M.

11. The process as claimed in one or more of claims 1 to 10, wherein the precursor of the insulins or of the insulin derivatives has the sequence according to the formula II
R²-R¹-(B2-B29)-Y-X-Gly-(A2-A20)-R³ (II),
in which
R² is
a) a hydrogen atom,
b) an amino acid residue from the group consisting of lysine (Lys) and arginine (Arg) or
c) a peptide having 2 to 45 amino acid residues, comprising the amino acid residue lysine (Lys) or arginine (Arg) at the carboxyl end of the peptide
R¹ is a phenylalanine residue (Phe) or a covalent bond,
(B2-B29) are the amino acid residues in the positions B2 to B29 of the B chain of human insulin, animal insulin or an insulin derivative which is optionally varied in one or more of these positions,
Y is a genetically encodable amino acid residue,
X is
a) an amino acid residue from the group consisting of lysine (Lys) and arginine (Arg) or
b) a peptide having 2 to 35 amino acid residues, comprising the amino acid residue lysine (Lys) or arginine (Arg) at the N-terminal end and at the carboxyl end of the peptide, or
c) a peptide having 2 to 35 genetically encodable amino acids, comprising 1 to 5 histidine residues,
(A2-A20)are the amino acid residues in the positions A2 to A20 of the B chain of human insulin, animal insulin or an insulin derivative which is optionally varied in one or more of these positions and
R³ is a genetically encodable amino acid residue.

12. The process as claimed in claim 11, wherein in formula II
R² is
a) a hydrogen atom or
b) a peptide having 2 to 25 amino acid residues, comprising the amino acid residue arginine (Arg) at the carboxyl end of the peptide,
R¹ is a phenylalanine residue (Phe),
(B2-B29) are the amino acid residues in the positions B2 to B29 of the B chain of human insulin,
Y is an amino acid residue from the group consisting of alanine (Ala), threonine (Thr) and serine (Ser),
X is the amino acid residue arginine (Arg) or a peptide having the amino acid sequence of the C chain of human insulin,
(A2-A20) are the amino acid residues in the positions A2 to A20 of the B chain of human insulin and
R³ is an amino acid residue from the group consisting of asparagine (Asn), serine (Ser) and glycine (Gly).

13. The process as claimed in claim 11, wherein in formula II
R² is
a) a hydrogen atom or
b) a peptide having 2 to 15 amino acid residues, at whose carboxyl end is found an arginine residue (Arg),
R¹ is a phenylalanine residue (Phe),
(B2-B29) are the amino acid residues in the positions B2 to B29 of the B chain of human insulin,
Y is a threonine residue (Thr),
X is the amino acid residue arginine (Arg) or a peptide having 2 to 35 amino acid residues, where at the beginning and at the end of the peptide there are two basic amino acid residues, in particular arginine (Arg) and/or lysine (Lys),
(A2-A20) are the amino acid residues in the positions A2 to A20 of the B chain of human insulin and
R³ is the amino acid residue asparagine (Asn) or glycine (Gly).

14. The process as claimed in one or more of claims 1 to 13, wherein an insulin or an insulin derivative of the formula I 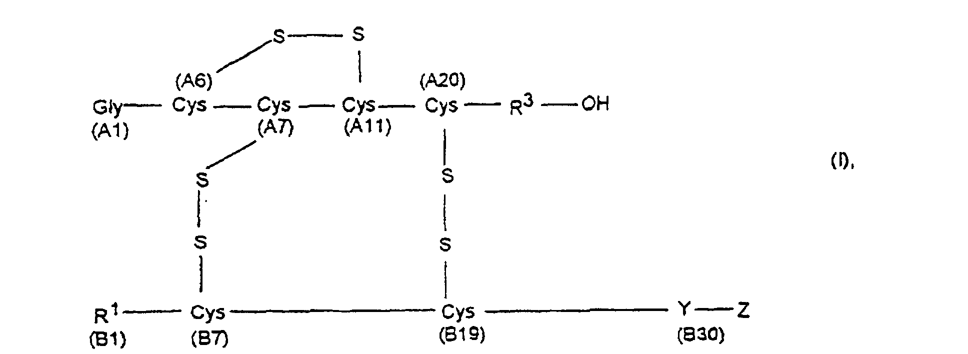 is obtained, in which the meanings are
Y a genetically encodable amino acid residue,
Z
a) an amino acid residue from the group consisting of His, Arg and Lys
b) a peptide having 2 or 3 amino acid residues comprising the amino acid residue Arg or Lys at the carboxyl end of the peptide,
c) a peptide having 2 - 35 genetically encodable amino acids, comprising 1 to 5 histidine residues, or
d) OH,
R¹ a phenylalanine residue (Phe)
R³ a genetically encodable amino acid residue,
where the residues A2 - A20 correspond to the amino acid sequence of the A chain of human insulin, animal insulin or an insulin derivative and the residues B2 - B29 correspond to the amino acid sequence of the B chain of human insulin, animal insulin or an insulin derivative.

15. The process as claimed in claim 14, wherein in formula I
Y is alanine (Ala), threonine (Thr) or serine (Ser)
Z is
a) arginine (Arg)
b) a dipeptide having 2 arginine residues (Arg-Arg)
c) OH,
R³ is asparagine (Asn), serine (Ser), Glycine (Gly).

16. The process as claimed in claim 14, wherein in formula I
Y is threonine (Thr)
Z is
a) arginine (Arg)
b) a dipeptide having 2 arginine residues (Arg-Arg) or 2 lysine residues (Lys-Lys)
c) OH,
R³ is asparagine (Asn), glycine (Gly).

17. The process as claimed in claim 15 or 16, wherein in formula I
Y is threonine (Thr)
Z is OH
R³ is asparagine (Asn).

18. The process as claimed in claim 15 or 16, wherein in formula I
Y is threonine (Thr)
Z is a dipeptide having 2 arginine residues (Arg-Arg)
R³ is glycine (Gly).

## Revendications

1. Procédé de préparation d'insulines ou d'un dérivé d'insuline avec des ponts de cystine liés correctement à partir d'un précurseur de l'insuline ou du dérivé d'insuline, dans lequel le précurseur est soumis, en présence de cystéine ou de chlorhydrate de cystéine et d'un adjuvant chaotrope, à un procédé de maturation, après lequel on obtient, à partir du précurseur par coupure enzymatique au moyen de trypsine ou d'une enzyme analogue à la trypsine et, de plus, éventuellement au moyen de carboxypeptidase B et ensuite d'une purification sur une résine absorbante, une insuline ou un dérivé de l'insuline avec des ponts de cystine liés correctement, **caractérisé en ce qu'**on effectue pour réaliser le procédé de maturation successivement les étapes suivantes consistant à :
(a) ajouter à une suspension aqueuse du précurseur de l'insuline ou du dérivé d'insuline une quantité de cystéine ou de chlorhydrate de cystéine, qui permet d'obtenir de 1 à 15 résidus SH de la cystéine ou du chlorhydrate de cystéine par groupe cystéine du précurseur,
(b) introduire la suspension du précurseur contenant de la cystéine ou du chlorhydrate de cystéine dans une solution de 4 à 9 fois molaire de l'adjuvant chaotrope à une valeur de pH d'environ 8 à environ 11,5 et à une température d'environ 15 à environ 55°C, maintenir le mélange obtenu pendant de 10 à 60 minutes environ à cette température et
(c) introduire le mélange à une valeur de pH d'environ 8 à environ 11,5 et à une température d'environ 5 à environ 30°C dans une quantité d'eau, qui permet d'obtenir une dilution de la concentration de la cystéine ou du chlorhydrate de cystéine dans le mélange à environ 1 à 5 mM et de l'adjuvant chaotrope à 0,2 à 1,0 M.

2. Procédé selon la revendication 1, **caractérisé en ce qu'**à l'étape (a) la quantité de cystéine ou de chlorhydrate de cystéine correspond à une quantité qui permet d'obtenir de 1 à 6 résidus SH de la cystéine ou du chlorhydrate de cystéine par groupe cystéine,
qu'à l'étape (b) on introduit la suspension du précurseur contenant de la cystéine ou du chlorhydrate de cystéine dans une solution 4 à 9 fois molaire de l'adjuvant chaotrope à une valeur de pH de 8 à 11 et à une température de 30 à 45°C, le maintien du mélange obtenu pendant 20 à 40 minutes à cette température et qu'à l'étape (c) on introduit le mélange à une valeur de pH de 8 à 11 et à une température de 15 à 20°C dans une quantité d'eau qui permet d'obtenir une dilution de la concentration de la cystéine ou du chlorhydrate de cystéine dans le mélange à environ 1 à 5 mM et une concentration de l'adjuvant chaotrope de 0,2 à 1,0 M.

3. Procédé selon la revendication 1 ou 2, **caractérisé en ce que** l'adjuvant chaotrope est la guanidine ou le chlorhydrate de guanidine.

4. Procédé selon la revendication 1 ou 2, **caractérisé en ce que** l'adjuvant chaotrope est l'urée.

5. Procédé selon une ou plusieurs des revendications 1 à 4, **caractérisé en ce que** la concentration de l'adjuvant chaotrope à l'étape (b) s'élève à une valeur située entre 7,0 et 9 M.

6. Procédé selon une ou plusieurs des revendications 1 à 5, **caractérisé en ce que** la température à l'étape (c) s'élève à 40°C.

7. Procédé selon une ou plusieurs des revendications 1 à 6, **caractérisé en ce que** la valeur de pH à l'étape (b) s'élève à une valeur située entre 10 et 11.

8. Procédé selon une ou plusieurs des revendications 1 à 7, **caractérisé en ce que** la valeur de pH à l'étape (c) s'élève à une valeur située entre 10 et 11.

9. Procédé selon une ou plusieurs des revendications 1 à 8, **caractérisé en ce qu'**à l'étape (c) la quantité d'eau permet d'obtenir une dilution de la concentration de la cystéine ou du chlorhydrate de cystéine dans le mélange de 2,5 à 3 mM et une concentration de l'adjuvant chaotrope de 0,5 M.

10. Procédé selon une ou plusieurs des revendications 2 à 9, **caractérisé en ce** la concentration de l'adjuvant chaotrope à l'étape (b) s'élève à 8 M, la température à l'étape (b) à 40°C, la valeur de pH à l'étape (b) à 10,6, la valeur de pH à l'étape (c) à 10,6 et à l'étape (c) la quantité d'eau permette d'obtenir une dilution de la concentration de la cystéine ou du chlorhydrate de cystéine dans le mélange à entre 2,5 et 3 mM et une concentration de l'adjuvant chaotrope de 0,5 M.

11. Procédé selon une ou plusieurs des revendications 1 à 10, **caractérisé en ce que** le précurseur des insulines ou des dérivés de l'insuline présente la séquence selon la formule générale II
R²-R¹- (B2-B29) -Y-X-Gly- (A2-A20) -R³ (II),
dans laquelle
R² représente
a) un atome d'hydrogène,
b) un résidu aminoacide choisi parmi le groupe de la lysine (Lys) ou de l'arginine (Arg) ou
c) un peptide avec 2 à 45 groupes aminoacides, contenant le résidu d'aminoacide lysine (Lys) ou arginine (Arg) à l'extrémité carboxyle du peptide
R¹ représente un groupe phénylalanine (Phe) ou une liaison covalente,
(B2-B29) représentent les groupes aminoacides dans les positions B2 à B29 de la chaîne B de l'insuline humaine, de l'insuline animale ou d'un dérivé d'insuline éventuellement modifié en une ou plusieurs de ces positions,
Y représente un groupe aminoacide génétiquement codable,
X représente
a) un groupe aminoacide choisi parmi le groupe de la lysine (Lys) ou de l'arginine (Arg) ou
b) un peptide avec 2 à 35 groupes aminoacides, contenant le groupe aminoacide lysine (Lys) ou arginine (Arg) à l'extrémité N-terminale et l'extrémité carboxyle du peptide, ou
c) un peptide avec 2 à 35 aminoacides génétiquement codables, contenant 1 à 5 groupes histidine,
(A2-A20) représentent les groupes aminoacides dans les positions A2 à A20 de la chaîne B de l'insuline humaine, de l'insuline animale ou d'un dérivé d'insuline éventuellement modifié en une ou plusieurs de ces positions et
R³ représente un groupe aminoacide génétiquement codable.

12. Procédé selon la revendication 11, **caractérisé en ce que** dans la formule II
R² représente
a) un atome d'hydrogène ou
b) un peptide avec 2 à 45 groupes aminoacides, contenant le groupe aminoacide arginine (Arg) à l'extrémité carboxyle du peptide
R¹ représente un groupe phénylalanine (Phe),
(B2-B29) représentent les groupes aminoacides dans les positions B2 à B29 de la chaîne B de l'insuline humaine,
Y représente un groupe aminoacide choisi parmi le groupe de l'alanine (Ala), de la thréonine (Thr) ou de la sérine (Ser),
X représente le groupe aminoacide arginine (Arg) ou un peptide avec la séquence d'aminoacide de la chaîne C de l'insuline humaine,
(A2-A20) représentent les groupes aminoacides dans les positions A2 à A20 de la chaîne B de l'insuline humaine et
R³ représente un groupe aminoacide choisi parmi le groupe de l'asparagine (Asn), de la sérine (Ser) ou de la glycine (Gly).

13. Procédé selon la revendication 11, **caractérisé en ce que** dans la formule II
R² représente
a) un atome d'hydrogène ou
b) un peptide avec 2 à 15 groupes aminoacides, à l'extrémité carboxyle duquel se trouve un groupe arginine (Arg),
R¹ représente un groupe phénylalanine (Phe),
(B2-B29) représentent les groupes aminoacides dans les positions B2 à B29 de la chaîne B de l'insuline humaine,
Y représente un groupe thréonine (Thr),
X représente le groupe aminoacide arginine (Arg) ou un peptide avec 2 à 35 groupes aminoacides, dans lequel au début et à la fin du peptide se trouvent deux groupes aminoacides basiques, en particulier l'arginine (Arg) et/ou la lysine (Lys),
(A2-A20) représentent les groupes aminoacides dans les positions A2 à A20 de la chaîne B de l'insuline humaine et
R³ représente le groupe aminoacide asparagine (Asn) ou glycine (Gly).

14. Procédé selon une ou plusieurs des revendications 1 à 13, **caractérisé en ce qu'**on obtient une insuline ou un dérivé d'insuline de formule I 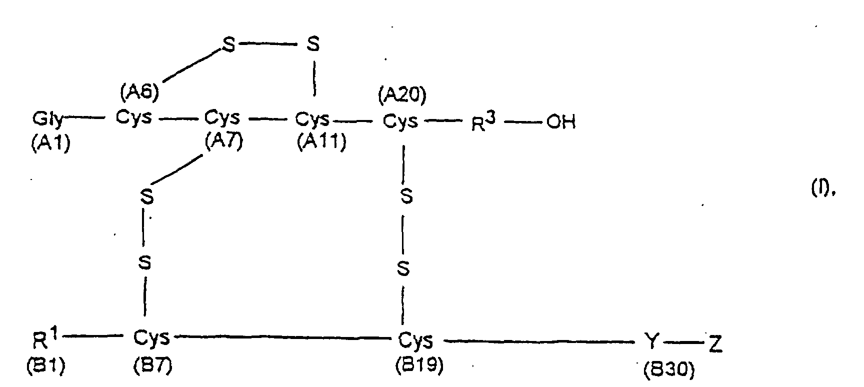 dans laquelle
Y représente un groupe aminoacide génétiquement codable,
Z représente
a) un groupe aminoacide choisi parmi le groupe His, Arg ou Lys,
b) un peptide avec 2 ou 3 groupes aminoacide, contenant le groupe aminoacide Arg ou Lys à l'extrémité carboxyle du peptide,
c) un peptide avec 2 à 35 aminoacides génétiquement codables, contenant de 1 à 3 groupes histidine, ou
d) OH,
R¹ représente un groupe phénylalanine (Phe)
R³ représente un groupe aminoacide génétiquement codable,
dans lesquels les groupes A2-A20 correspondent à la séquence d'acides aminés de la chaîne A de l'insuline humaine, de l'insuline animale ou d'un dérivé de l'insuline et les groupes B2-B29 à la séquence d'acides aminés de la chaîne B de l'insuline humaine, de l'insuline animale ou d'un dérivé de l'insuline.

15. Procédé selon la revendication 14, **caractérisé en ce que** dans la formule I
Y représente l'alanine (Ala), la thréonine (Thr) ou la sérine (Ser)
Z représente
a) l'arginine (Arg)
b) un dipeptide avec 2 groupes arginine (Arg-Arg)
c) OH,
R³ représente l'asparagine (Asn), la sérine (Ser), la glycine (Gly).

16. Procédé selon la revendication 14, **caractérisé en ce que** dans la formule I
Y représente la thréonine (Thr)
Z représente
a) l'arginine (Arg)
b) un dipeptide avec 2 groupes arginine (Arg-Arg) ou 2 groupes lysine (Lys-Lys)
c) OH,
R³ représente l'asparagine (Asn), la glycine (Gly).

17. Procédé selon la revendication 15 ou 16, **caractérisé en ce que** dans la formule I
Y représente la thréonine (Thr)
Z représente OH,
R³ représente l'asparagine (Asn).

18. Procédé selon la revendication 15 ou 16, **caractérisé en ce que** dans la formule I
Y représente la thréonine (Thr)
Z représente un dipeptide avec deux groupes arginine (Arg-Arg)
R³ représente la glycine (Gly).
